# EUROPEAN PATENT APPLICATION

(11) **EP 4 186 530 A1**
(43) Date of publication of application: **31.05.2023**
(21) Application number: 21211170.2
(22) Date of filing: 29.11.2021
(51) Int. Cl.: A61L 2/10, B60Q 3/252, B60Q 3/68, B60Q 3/208

(54) **A DISINFECTING INTERIOR VEHICLE LIGHTING SYSTEM AND A METHOD FOR OPERATING AN INTERIOR VEHICLE LIGHTING SYSTEM**

(71) Applicant: Ningbo Geely Automobile Research & Development Co. Ltd., Hangzhou Bay New District Ningbo 315336 (CN); Zhejiang Geely Holding Group Co., Ltd., Hangzhou, Zhejiang 310051 (CN)
(72) Inventor: JOHANSSON, Jonathan, 417 14 GÖTEBORG (SE); GHATUARI, Sakti Ranjan, 417 21 GÖTEBORG (SE)
(74) Representative: Zacco Sweden AB

(57) **Abstract**

An interior vehicle lighting system (LS) and method providing a disinfecting effect to interior surfaces (S) of a vehicle (V), where the lighting system (LS) comprises at least one light source (1) attached to an interior sunscreen cover (2). The at least one light source (1) is adapted to emit light with a disinfecting effect to the interior surfaces (S) of the vehicle (V), and the interior sunscreen cover (2) is movably arranged relative to a sunroof (3) of the vehicle (V). The at least one light source (1) is arranged for being displaced relative to the interior surfaces (S) of the vehicle (V) upon movement of the interior sunscreen cover (2).

## Description

### TECHNICAL FIELD

The present disclosure relates to an interior vehicle lighting system providing a disinfecting effect to interior surfaces of a vehicle. The lighting system comprises at least one light source used for providing the disinfecting effect. The disclosure further relates to a method for operating an interior vehicle lighting system.

### BACKGROUND

To use disinfecting liquids or UV light to disinfect hands or surfaces are well-known methods commonly used today. Especially during pandemic situations, more and more people become aware of the negative effects from virus and bacteria, and there is therefore a need for disinfecting not only hands or surfaces at home, but also interior surfaces of vehicles. Further, there is a growing need for disinfecting vehicles in the future, when vehicles are shared between many different users, such as in taxis, rental vehicles, car-sharing vehicles, or in shared autonomous drive vehicles. Methods used today for disinfecting vehicle interior surfaces are often troublesome and many times only certain parts of the vehicle interior surfaces are properly disinfected, for example when using disinfecting liquids or UV light.

There is thus a need for an improved way of disinfecting interior surfaces of vehicles that is simple and efficient in order to provide a desired result.

### SUMMARY

An object of the present disclosure is to provide an interior vehicle lighting system providing a disinfecting effect to interior surfaces of a vehicle, and a method for operating an interior vehicle lighting system, where the previously mentioned problems are avoided. This object is at least partly achieved by the features of the independent claims. The dependent claims contain further developments of the interior vehicle lighting system and the method for operating an interior vehicle lighting system.

The disclosure concerns an interior vehicle lighting system providing a disinfecting effect to interior surfaces of a vehicle. The lighting system comprises at least one light source attached to an interior sunscreen cover, and the at least one light source is adapted to emit light with a disinfecting effect to the interior surfaces of the vehicle. The interior sunscreen cover is movably arranged relative to a sunroof of the vehicle, and the at least one light source is arranged for being displaced relative to the interior surfaces of the vehicle upon movement of the interior sunscreen cover.

Advantages with these features are that through the movable arrangement of the at least one light source attached to the interior sunscreen cover, the disinfecting light can be easily distributed to different interior surfaces of the vehicle by displacement of the interior sunscreen cover. Many vehicle models are already today equipped with a sunroof and an interior sunscreen cover that fully or partly is preventing light, such as sunlight, from entering the interior compartment of the vehicle via the sunroof. The interior sunscreen covers are commonly movable and can be displaced between different positions to allow or prevent light from entering the interior compartment. The solution is providing an improved way of disinfecting the interior surfaces that is simple and efficient for a desired result, compared to traditional ways of disinfecting vehicle interior surfaces where only certain parts of the vehicle interior surfaces are properly disinfected. The growing future need for disinfecting vehicle interior surfaces can be met by the solution by attaching the at least one light source to the interior sunscreen cover.

According to an embodiment, the at least one light source comprises a UV emitting lamp, where the at least one light source is adapted to emit UV light to the interior surfaces of the vehicle. UV light is suitable for vehicle disinfecting applications, and the use of a UV emitting lamp is providing an efficient way of disinfecting the interior vehicle surfaces. Any suitable type of UV emitting lamp may be used. One example of UV emitting lamps are light emitting diodes (LED) adapted for emitting UV light.

According to an embodiment, the at least one light source is forming an integrated structural part of the interior sunscreen cover. By integrating the at least one light source as a structural part of the interior sunscreen cover, an efficient and aesthetically attractive solution is achieved. The at least one light source may be arranged in a suitable position or in suitable positions integrated in the interior sunscreen cover for an efficient light distribution, both upon movement of the interior sunscreen cover and when stationary.

According to an embodiment, the vehicle lighting system further comprises a track arrangement, where the track arrangement is adapted for guiding the interior sunscreen cover upon movement. The track arrangement is suitably arranged in connection to a roof structure of the vehicle on opposite lateral sides of the sunroof construction of the vehicle. The track arrangement has the purpose to steer and control the position of the interior sunscreen cover when stationary and upon movement relative to the roof construction of the vehicle.

According to an embodiment, the interior sunscreen cover comprises a leading edge. The at least one light source is attached to the interior sunscreen cover in connection to the leading edge. The track arrangement comprises guiding members adapted for directing a leading edge section of the interior sunscreen cover including the leading edge into a downwards direction away from the track structure, where the at least one light source is displaced in the downwards direction. The guiding members are allowing the leading edge section with the leading edge to depart from the track structure into a downwards direction for efficiently disinfecting the interior vehicle surfaces. By the downwards movement, the at least one light source can emit light onto surfaces that are otherwise blocked from light emitted by the at least one light source.

According to an embodiment, the leading edge is extending in a lateral vehicle direction. The extension in the lateral direction is suitable when the track structure is extending in a longitudinal vehicle direction, and the leading edge is suitably extending between two parallel tracks for a simple and efficient displacement of the interior sunscreen cover with the at least one light source relative to the interior surfaces of the vehicle.

The disclosure further concerns a method for operating an interior vehicle lighting system providing a disinfecting effect to interior surfaces of a vehicle. The lighting system comprises at least one light source attached to an interior sunscreen cover, and the interior sunscreen cover is movably arranged relative to a sunroof of the vehicle. The method comprises the steps: activating the at least one light source; displacing the at least one light source relative to the interior surfaces of the vehicle upon movement of the interior sunscreen cover; and emitting light with a disinfecting effect to the interior surfaces of the vehicle with the at least one light source.

Advantages with this method are that by moving the at least one light source attached to the interior sunscreen cover, the disinfecting light can be easily distributed to different interior surfaces of the vehicle by displacement of the interior sunscreen cover. The solution is providing an improved way of disinfecting the interior surfaces that is simple and efficient for a desired result, compared to traditional ways of disinfecting vehicle interior surfaces where only certain parts of the vehicle interior surfaces are properly disinfected.

According to an embodiment, the at least one light source comprises a UV emitting lamp, wherein the method further comprises the step: emitting UV light to the interior surfaces of the vehicle with the at least one light source. UV light is suitable for vehicle disinfecting applications, and by emitting UV light from the UV emitting lamp the interior vehicle surfaces are disinfected in a simple and efficient way.

According to an embodiment, the method further comprises the steps: initiating an activating command for moving the interior sunscreen cover, where upon initiation of the activating command, the at least one light source is displaced with the interior sunscreen cover from an initial position. The activating command is thus used as an initiation for moving the interior sunscreen cover from an initial position and the activating command can be seen as the initiation of the displacement of the interior sunscreen cover from the initial position. The initial position may be any suitable pre-determined starting position from which the interior sunscreen cover is displaced, and the activating command is for example triggered by a user of the vehicle with a suitable initiating means that is initiating the disinfecting process.

According to an embodiment, the method further comprises the step: displacing the at least one light source with the interior sunscreen cover from the initial position to at least one stopping position. Once the interior sunscreen cover has reached the stopping position, the interior sunscreen cover is stopped from further movement. The stopping position may for example be used for extending the time for emitting light in a certain position. The stopping position may thereafter be regarded as a new initial position for a following operational sequence.

According to an embodiment, the method further comprises the steps: displacing the at least one light source with the interior sunscreen cover from the initial position upon initiation of the activation command, and stop the displacement of the at least one light source with the interior sunscreen cover upon initiation of a stopping command. The stopping command may be initiated by the user or alternatively the stopping command is triggered at a pre-determined stopping position by for example a control unit of the vehicle or a separately arranged control unit for the interior vehicle lighting system.

According to an embodiment, the stopping command is initiated after a predetermined time duration. In this embodiment, the stopping command is suitably triggered by the control unit after a predetermined time from the initiation of the activating command.

According to an embodiment, the at least one light source is moving from the initial position in a longitudinal vehicle direction. This orientation and moving direction is suitable when the interior sunscreen cover is configured for being displaced in the longitudinal vehicle direction, and suitable for emitting light to a major part of the interior vehicle surfaces.

According to an embodiment, the vehicle lighting system further comprises a track arrangement adapted for guiding the interior sunscreen cover upon movement. The interior sunscreen cover comprises a leading edge and a leading edge section including the leading edge. The at least one light source is attached to the interior sunscreen cover in connection to the leading edge, and the track arrangement comprises guiding members. The method further comprises the step: displacing the leading edge section and the at least one light source in a downwards direction away from the track structure by means of the guiding members. The track arrangement is suitably arranged in connection to the roof structure of the vehicle on opposite lateral sides of the sunroof construction of the vehicle. The track arrangement has the purpose to steer and control the position of the interior sunscreen cover when stationary and upon movement relative to the roof construction of the vehicle. The guiding members are allowing the leading edge section with the leading edge to depart from the track structure by the displacement in the downwards direction for efficiently disinfecting the interior vehicle surfaces. By the downwards displacement, the at least one light source can emit light onto surfaces that are otherwise blocked from light emitted by the at least one light source.

According to an embodiment, the method further comprises the step: displacing the leading edge section and the at least one light source of the interior sunscreen cover in an upwards direction after displacing the leading edge section and the at least one light source in the downwards direction for returning the leading edge to the track structure. The displacement in the upwards direction is providing an efficient way of returning the leading edge to the track structure.

### BRIEF DESCRIPTION OF DRAWINGS

The disclosure will be described in detail in the following, with reference to the attached drawings, in which
- Fig. 1: shows schematically, in an external perspective view from above, a vehicle with an interior vehicle lighting system, according to the disclosure,
- Fig. 2: shows schematically, in an internal perspective view from below, an interior compartment of the vehicle with the interior vehicle lighting system according to the disclosure,
- Fig. 3a-e: show schematically, in side views, the vehicle with embodiments of the interior vehicle lighting system in different operating positions, according to the disclosure,
- Fig. 4a-c: show schematically, in side views, an embodiment of a track system for the interior vehicle lighting system according to the disclosure, and
- Fig. 5: shows a flowchart of exemplified method steps for operation of the interior vehicle lighting system, according to the disclosure.

### DESCRIPTION OF EXAMPLE EMBODIMENTS

Various aspects of the disclosure will hereinafter be described in conjunction with the appended drawings to illustrate and not to limit the disclosure, wherein like designations denote like elements, and variations of the described aspects are not restricted to the specifically shown embodiments, but are applicable on other variations of the disclosure.

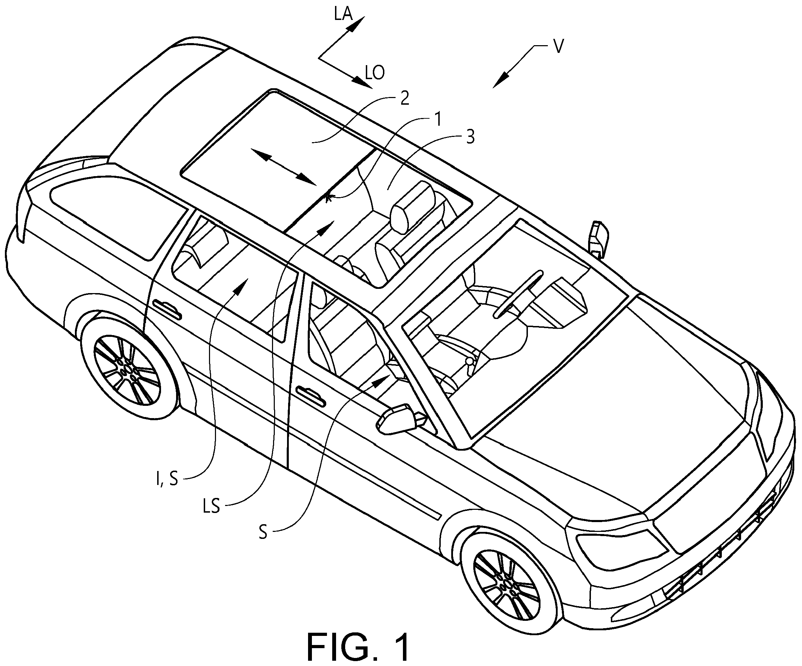

Figures 1-2 schematically show a vehicle V with a sunroof 3 arranged in a roof structure of the vehicle V. The sunroof 3 may be of any adequate construction and configuration, and suitably the sunroof 3 is made of a glass structure or other light transmitting material allowing light to enter an interior compartment I of the vehicle V. The vehicle V may be of any type and in the illustrated embodiments a car is schematically shown. It should however be understood that the sunroof 3 instead may be arranged in a bus, truck or other type of vehicle.

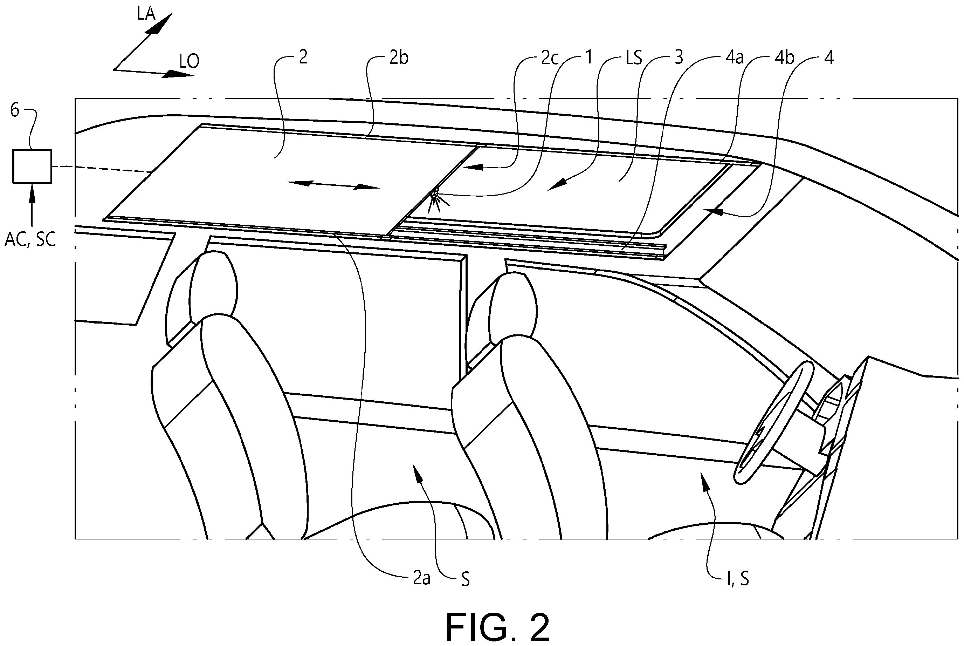

To prevent occupants from light entering the sunroof 3, an interior sunscreen cover 2 is arranged in connection to the sunroof 3 on the inner side of the sunroof 3 as shown in figure 2. The interior sunscreen cover 2 is movably arranged relative to the sunroof 3 and suitably the interior sunscreen cover 2 is movable in a longitudinal vehicle direction LO between different positions, where the interior sunscreen cover 2 can be positioned to fully cover the sunroof 3, partly cover the sunroof 3, or alternatively positioned not to cover the sunroof 3. The interior sunscreen cover 2 may be movably connected to a track arrangement 4, where the track arrangement 4 is adapted for guiding the interior sunscreen cover 2 upon movement. The interior sunscreen cover 2 may be made of any suitable material, and the material may be configured to fully block or partly block light from entering the interior compartment I.

The vehicle V is further arranged with an interior vehicle lighting system LS that is providing a disinfecting effect to interior surfaces S of the vehicle V. The lighting system LS comprises at least one light source 1 attached to the interior sunscreen cover 2. The at least one light source 1 is adapted to emit light with a disinfecting effect to the interior surfaces S of the vehicle V. Due to the movable arrangement of the interior sunscreen cover 2 relative to the sunroof 3, the at least one light source 1 is being displaced relative to the interior surfaces S of the vehicle V upon movement of the interior sunscreen cover 2. With the expression interior surfaces S is meant surfaces within the interior compartment I of the vehicle V, such as, but not limited to, dashboard and trim panel surfaces, vehicle seat surfaces, steering wheel surfaces, gear selector and centre console surfaces, and floor surfaces. It should be understood that any suitable number of light sources could be attached to the interior sunscreen cover 2, depending on the design and construction of the lighting system LS. The light sources may be arranged in any suitable position on the interior sunscreen cover 2 for an efficient disinfection of the interior surfaces S. The light source 1 may be configured to direct light in many different directions or controlled to direct light in certain desired directions only.

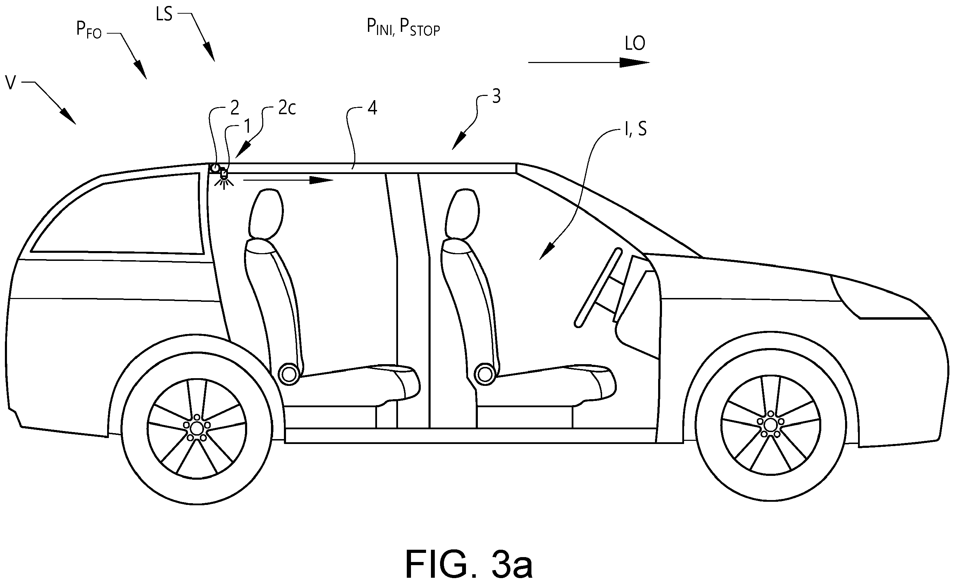

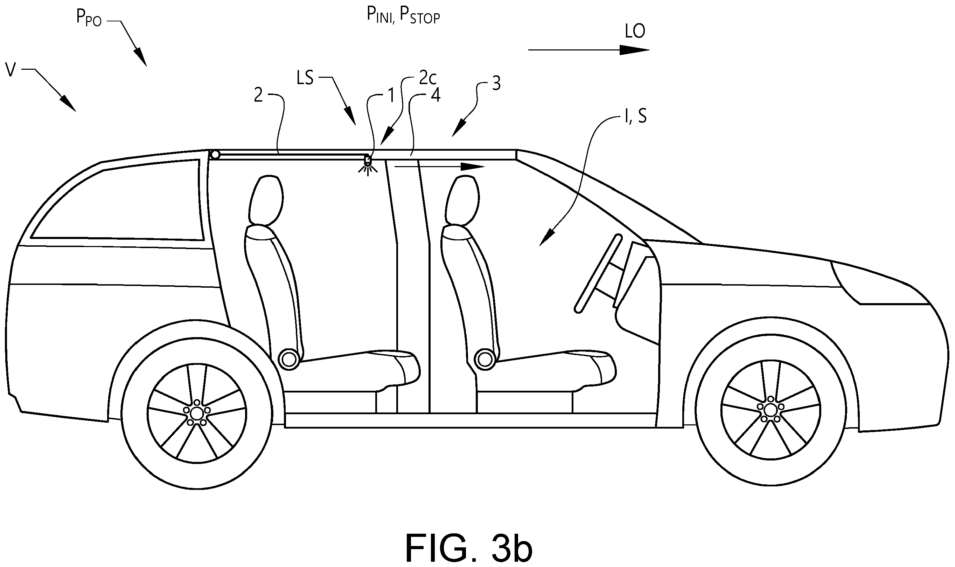

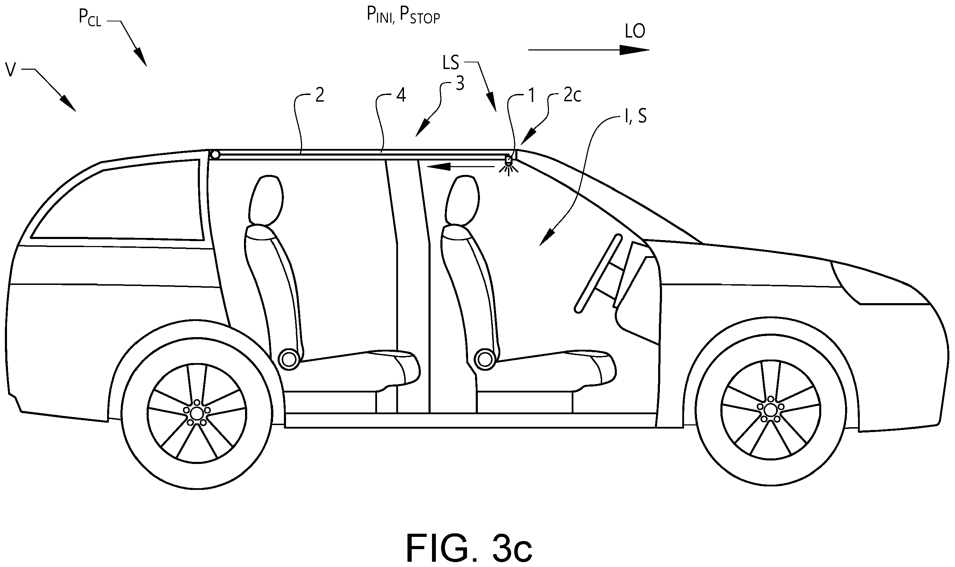

In figures 3a-c, an embodiment of an interior vehicle lighting system LS is schematically illustrated wherein the lighting system LS comprises a light source 1 attached to an interior sunscreen cover 2 arranged in connection to a sunroof 3. The interior sunscreen cover 2 is movably arranged relative to the sunroof 3 of the vehicle V in a longitudinal vehicle direction LO between different positions. The interior sunscreen cover 2 can be positioned to fully cover the sunroof 3 in a closed position P_{CL} as shown in figure 3c, partly cover the sunroof 3 in a partly open position P_{PO} as shown in figure 3b, or alternatively positioned not to cover the sunroof 3 in a fully open position P_{FO} as shown in figure 3a. The interior sunscreen cover 2 is suitably configured to be positioned in any position between the closed position P_{CL} and the fully open position P_{FO}, and the positioning of the interior sunscreen cover 2 is for example established by an actuator, such as an electric motor, where the actuator is controlling the movement of the interior sunscreen cover via wires, gears or other suitable means. The actuator may be connected to a control unit 6 for controlling the movement of the interior sunscreen cover 2. The control unit 6 may form part of or be integrated with the control unit or control units of the vehicle V, or alternatively configured as a separate control unit for the lighting system LS. In the partly open position P_{PO} and the fully open position P_{FO}, the interior sunscreen cover 2 may at least partly be arranged in a rolled-up or deflected configuration as well known in the art.

The interior vehicle lighting system LS is as described above used for providing a disinfecting effect to interior surfaces S of the vehicle V, such as, but not limited to, dashboard and trim panel surfaces, vehicle seat surfaces, steering wheel surfaces, gear selector and centre console surfaces, and floor surfaces. The light source 1 is attached to the interior sunscreen cover 2 and adapted to emit light with a disinfecting effect to the interior surfaces S.

The light source 1 may be arranged as a light source unit that is attached to the interior sunscreen cover 2 with suitable attachment means. The light source 1 may alternatively be integrated in the interior sunscreen cover 2, and in this way, the light source 1 is forming an integrated structural part of the interior sunscreen cover 2. By integrating the light source 1 as a structural part of the interior sunscreen cover, an efficient and aesthetically attractive design can be achieved. The light source 1 is arranged in a suitable position attached to or integrated in the interior sunscreen cover for an efficient light distribution, both upon movement of the interior sunscreen cover and when stationary. The light source 1 is for example powered by the vehicle V and connected to a vehicle electric system via electric wires. Alternatively, the light source 1 is powered by a battery unit or other power means. The light source may be connected to and controlled by the control unit 6.

The interior sunscreen cover 2 is movably arranged relative to the sunroof 3 of the vehicle V. In this way, the light source 1 is displaced relative to the interior surfaces S upon movement of the interior sunscreen cover 2. Thus, the light source 1 is moving with the interior sunscreen cover 2 when the interior sunscreen cover 2 is displaced relative to the sunroof 3. Through the movable arrangement of the light source 1 attached to the interior sunscreen cover 2, the disinfecting light can be easily distributed to different interior surfaces S of the vehicle V by displacement of the interior sunscreen cover 2.

The light source 1 may comprise a UV emitting lamp, and by the UV emitting lamp the light source 1 is emitting UV (ultraviolet) light to the interior surfaces S of the vehicle V. UV light is suitable for vehicle disinfecting applications, and the UV emitting lamp is thus used for disinfecting the interior vehicle surfaces in a convenient manner. UV light, including UVC light with a wavelength between 200 and 280 nanometres, has a long history as a proven disinfectant. UV light is for example used for disinfecting vehicles, hospital and laboratory spaces, as well as businesses including airlines, hotels, and retail spaces. Any suitable type of UV emitting lamp may be used. One example of UV emitting lamps are light emitting diodes (LED) adapted for emitting UV light, also called UV LED. UVC LED is suitably used for an efficient disinfection. The light source 1 is suitably arranged to emit the UV light in different directions for an efficient disinfection of the interior surfaces S. The positioning of the light source 1 on the interior sunscreen cover 2 is also chosen for an efficient light distribution.

The control unit 6 may be programmed for specific displacement patterns of the interior sunscreen cover 2 during a disinfecting operation. The interior sunscreen cover 2 may for example be moved in reciprocating displacement patterns for an efficient disinfection of the interior surfaces S, as indicated with the double arrows in figures 1 and 2.

As shown in figure 2, the vehicle lighting system LS further comprises a track arrangement 4, and the track arrangement 4 is adapted for guiding the interior sunscreen cover 2 upon movement. The track arrangement in the illustrated embodiment comprises a first track structure 4a and a second track structure 4b, each having an extension in the longitudinal vehicle direction LO. The first track structure 4a and the second track structure 4b are arranged in a parallel or essentially parallel relationship and spaced apart in a lateral vehicle direction LA, as understood from figure 2, and the interior sunscreen cover 2 is configured for extending between the first track structure 4a and the second track structure 4b. A first lateral side 2a of the interior sunscreen cover 2 is configured for engaging the first track structure 4a, and a second lateral side 2b of the interior sunscreen cover 2 is configured for engaging the second track structure 4b. The first lateral side 2a and the second lateral side 2b may each be provided with guiding means or connection members that are running in the respective track structures for positioning the lateral sides of the interior sunscreen cover 2 into the track structures when the interior sunscreen cover 2 is displaced or at rest.

As shown in for example figure 2, the interior sunscreen cover 2 comprises a leading edge 2c, and in the illustrated embodiment, the light source 1 is attached to the interior sunscreen cover 2 in connection to the leading edge 2c. With the positioning of the light source 1 in connection to the leading edge 2c, the light source 1 can emit light onto a large interior surface area of the interior compartment I upon movement of the interior sunscreen cover 2, as will be further described below. The leading edge 2c is extending between the first track structure 4a and the second track structure 4b and has thus an extension in, or essentially in, the lateral vehicle direction LA. The leading edge 2c may have a straight configuration, or a shaped configuration, such as for example a slightly curved shape, depending on the design of the interior sunscreen cover 2.

In the embodiment illustrated in figure 3c, the interior sunscreen cover 2 is positioned to cover the sunroof 3 in the closed position P_{CL}. In the closed position P_{CL}, the leading edge 2c is arranged in a forward position of the interior compartment I. This position of the interior sunscreen cover 2 is used for blocking light, such as sunlight, from entering the interior compartment I of the vehicle V via the sunroof 3. If the interior sunscreen cover 2 is at rest in the closed position P_{CL}, this position may be an initial position P_{INI} from which a disinfecting operation is started. When the interior sunscreen cover 2 is displaced from the closed position P_{CL}, the leading edge 2c is moved in the longitudinal direction LO rearwards, as indicated with the arrow in figure 3c. In figure 3a, the interior sunscreen cover 2 is positioned not to cover the sunroof 3 in the fully open position P_{FO}. In the fully open position P_{FO}, the leading edge 2c is arranged in a rearward position of the interior compartment I. This position of the interior sunscreen cover 2 is used for allowing light, such as sunlight, to enter the interior compartment I of the vehicle V via the sunroof 3. If the interior sunscreen cover 2 is at rest in the fully open position P_{FO}, this position may be an alternative initial position P_{INI} from which a disinfecting operation is started. When the interior sunscreen cover 2 is displaced from the fully open position P_{FO}, the leading edge 2c is moved in the longitudinal direction LO forwards, as indicated with the arrow in figure 3a. It should be understood that any suitable positioning of the interior sunscreen cover 2 could be an initial position P_{INI} for a disinfecting operation, such as the closed position P_{CL}, the fully open position P_{FO}, or any position between the closed position P_{CL} and the fully open position P_{FO} defined above as the partly open position P_{PO}.

To displace the interior sunscreen cover 2, a user of the vehicle V may manually initiate the movement of the interior sunscreen cover 2 via a switch button or other suitable activating means arranged inside the interior compartment I. During normal vehicle use, the light source 1 is turned off and the interior sunscreen cover 2 is positioned by the user in any desired position for allowing light into the interior compartment I through the sunroof 3, or for fully or partly blocking light from entering the interior compartment I through the sunroof 3.

To initiate a disinfecting operation, an activating command AC may first be initiated by the user for moving the interior sunscreen cover 2 with the attached or integrated light source 1. The activating command AC may for example be initiated by the user through a suitable interface in the vehicle V, such as a push button, or a human machine interface (HMI), such as a control panel, arranged in the interior compartment I. Alternatively, the activating command AC may be initiated by the user via a remote device connected to or associated with the vehicle V, such as a smartphone or a remote key device. In yet another embodiment, the control unit 6 is initiating the activating command AC based on pre-programmed criteria, for example based on the number of different occupants that have been travelling in the vehicle or based on a time duration from the previous disinfecting operation. The activating command AC may trigger different disinfecting operations, such as disinfection of all accessible interior surfaces S, or if for example only the front seats of the vehicle have been used since the last disinfecting operation only the interior surfaces S in connection to the front seats are disinfected. Different disinfecting operation sequences may be selected by the user. For a safe disinfection operation, the vehicle V may be equipped with suitable sensors that are detecting that no occupants are present in the vehicle when the disinfecting operation is taking place.

Once the activating command AC has been initiated, the light source 1 is activated and displaced relative to the interior surfaces S of the vehicle V upon movement of the interior sunscreen cover 2. The activation and displacement of the light source 1 is controlled by the control unit 6. Through the activation and displacement of the light source 1, light with a disinfecting effect is emitted to the interior surfaces S of the vehicle V from the light source 1.

As described above, the interior sunscreen cover 2 can be positioned to fully cover the sunroof 3 in the closed position P_{CL} as shown in figure 3c, partly cover the sunroof 3 in a partly open position P_{PO} as shown in figure 3b, or alternatively positioned not to cover the sunroof 3 in a fully open position P_{FO} as shown in figure 3a. The interior sunscreen cover 2 may be positioned in any position when the activating command AC is initiated. Thus, the interior sunscreen cover 2 may be positioned in the closed position P_{CL}, the fully open position P_{FO}, or in the partly open position P_{PO} between the closed position P_{CL} and the fully open position P_{FO}, when the activating command AC is initiated. The position in which the light source 1 is positioned when the activating command AC is initiated is defined as the initial position P_{INI} for the disinfecting operation. Upon initiation of the activating command AC for moving the interior sunscreen cover 2, the light source 1 is displaced with the interior sunscreen cover 2 from the initial position P_{INI}.

If the activating command AC is initiated when the interior sunscreen cover 2 is positioned in the closed position P_{CL}, as shown in figure 3c, the closed position P_{CL} is the initial position P_{INI} of the light source 1 from which the disinfecting operation is started. In this case, the interior sunscreen cover 2 is displaced towards the fully open position P_{FO}, and upon displacement of the interior sunscreen cover 2 the activated light source 1 is emitting light with the disinfecting effect onto the interior surfaces S of the vehicle V. When the interior sunscreen cover 2 with the light source 1 upon displacement reaches the fully open position P_{FO}, the interior sunscreen cover 2 with the light source 1 may be displaced back towards the closed position P_{CL}. When the interior sunscreen cover 2 with the light source 1 upon displacement reaches the closed position P_{CL}, the interior sunscreen cover 2 with the light source 1 may again be displaced towards the fully open position P_{FO} for another displacement cycle. With this operational configuration, the interior sunscreen cover 2 with the light source 1 may be displaced in a reciprocating manner between the closed position P_{CL} and the fully open position P_{FO} for an efficient disinfecting operation of the interior surfaces S. The number of operating cycles may be determined by the user or as a pre-defined operation by the control unit 6. It should be understood that the interior sunscreen cover 2 with the light source 1 in an alternative embodiment could be displaced from the closed position P_{CL} as the initial position P_{INI} to any position between the closed position P_{CL} and the fully open position P_{FO} and back to the closed position P_{CL} for an alternative operational cycle.

If the activating command AC is initiated when the interior sunscreen cover 2 is positioned in the fully open position P_{FO}, as shown in figure 3a, the fully open position P_{FO} is the initial position P_{INI} of the light source 1 from which the disinfecting operation is started. In this case, the interior sunscreen cover 2 is displaced towards the closed position P_{CL}, and upon displacement of the interior sunscreen cover 2 the activated light source 1 is emitting light with the disinfecting effect onto the interior surfaces S of the vehicle V. When the interior sunscreen cover 2 with the light source 1 upon displacement reaches the closed position P_{CL}, the interior sunscreen cover 2 with the light source 1 may be displaced back towards the fully open position P_{FO}. When the interior sunscreen cover 2 with the light source 1 upon displacement reaches the fully open position P_{FO}, the interior sunscreen cover 2 with the light source 1 may again be displaced towards the closed position P_{CL} for another displacement cycle. With this operational configuration, the interior sunscreen cover 2 with the light source 1 may be displaced in a reciprocating manner between the fully open position P_{FO} and the closed position P_{CL} for an efficient disinfecting operation of the interior surfaces S. The number of operating cycles may be determined by the user or as a pre-defined operation by the control unit 6. It should be understood that the interior sunscreen cover 2 with the light source 1 in an alternative embodiment could be displaced from the fully open position P_{FO} as the initial position P_{INI} to any position between the fully open position P_{FO} and the closed position P_{CL} and back to the fully open position P_{FO} for an alternative operational cycle.

If the activating command AC is initiated when the interior sunscreen cover 2 is positioned in the partly open position P_{PO}, as shown in figure 3b, and thus in any position between the closed position P_{CL} and the fully open position P_{FO}, the partly open position P_{PO} is the initial position P_{INI} of the light source 1 from which the disinfecting operation is started. In this case, the interior sunscreen cover 2 is displaced towards the closed position P_{CL} or towards the fully open position P_{FO}, and upon displacement of the interior sunscreen cover 2 the activated light source 1 is emitting light with the disinfecting effect onto the interior surfaces S of the vehicle V. When the interior sunscreen cover 2 with the light source 1 upon displacement reaches the closed position P_{CL} or the fully open position P_{FO}, the interior sunscreen cover 2 with the light source 1 may be displaced back towards the opposite position. The interior sunscreen cover 2 with the light source 1 may be operated as described above in displacement cycles, and with such an operational configuration, the interior sunscreen cover 2 with the light source 1 may be displaced in a reciprocating manner from the initial position P_{INI} towards the closed position P_{CL} and thereafter between the closed and fully open positions, or the interior sunscreen cover 2 with the light source 1 may be displaced in a reciprocating manner from the initial position P_{INI} towards the fully open position P_{FO} and thereafter between the fully open and closed positions, for an efficient disinfecting operation of the interior surfaces S. The number of operating cycles may be determined by the user or as a pre-defined operation by the control unit 6. It should be understood that the interior sunscreen cover 2 with the light source 1 in an alternative embodiment could be displaced from the partly open position P_{PO} as the initial position P_{INI} to any other partly open position P_{PO} between the fully open position P_{FO} and the closed position P_{CL} and back to the initial position P_{INI} for an alternative operational cycle.

In the embodiment illustrated in figures 3a-c, the light source 1 is moving from the initial position P_{INI} in a longitudinal vehicle direction LO. It should however be understood that the lighting system may be arranged in other suitable ways, where the light source is moving in directions different from the longitudinal vehicle direction LO.

The operational cycle could be programmed in any suitable way depending on the design and configuration of the interior surfaces S of the vehicle V. As an example, the interior sunscreen cover 2 with the light source 1 may be displaced in a random displacement pattern or in a displacement pattern where a specific part or specific parts of the interior compartment I receive more emitted light from the light source 1 than other interior surfaces S. If there for example is a specific need for disinfecting the steering wheel, the interior sunscreen cover 2 with the light source may be stopped for a specific time duration in a suitable position or in suitable positions for emitting light towards the steering wheel.

When disinfecting operation is completed, the displacement of the interior sunscreen cover 2 is stopped with the light source 1 in a stopping position P_{STOP}. If the displacement is intermittent, there may be more than one stopping position P_{STOP} during the disinfecting operation. Thus, the light source 1 is displaced with the interior sunscreen cover 2 from the initial position P_{INI} to at least one stopping position P_{STOP}. In one example embodiment, the light source 1 is displaced with the interior sunscreen cover 2 from the initial position P_{INI} upon initiation of the activation command AC, and the displacement of the light source 1 with the interior sunscreen cover 2 is stopped in the stopping position P_{STOP} upon initiation of a stopping command SC. The stopping command SC could be initiated by the user or by the control unit 6. The stopping command SC may for example be initiated by the user through a suitable interface in the vehicle V, such as a push button, or a human machine interface (HMI), such as a control panel, arranged in the interior compartment I. Alternatively, the stopping command SC may be initiated by the user via a remote device connected to or associated with the vehicle V, such as a smartphone or a remote key device. In yet another alternative, the control unit 6 is initiating the stopping command SC based on pre-programmed criteria, such as a predetermined time duration.

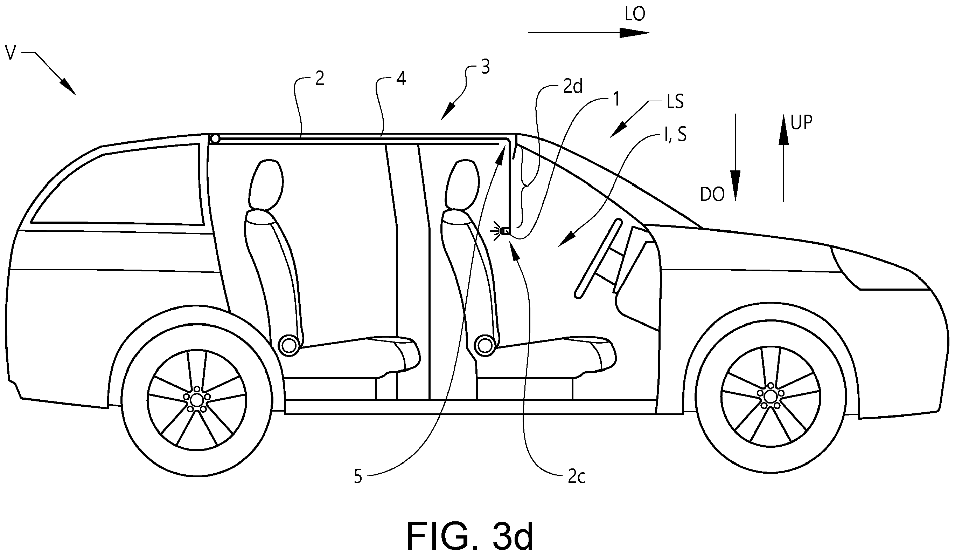

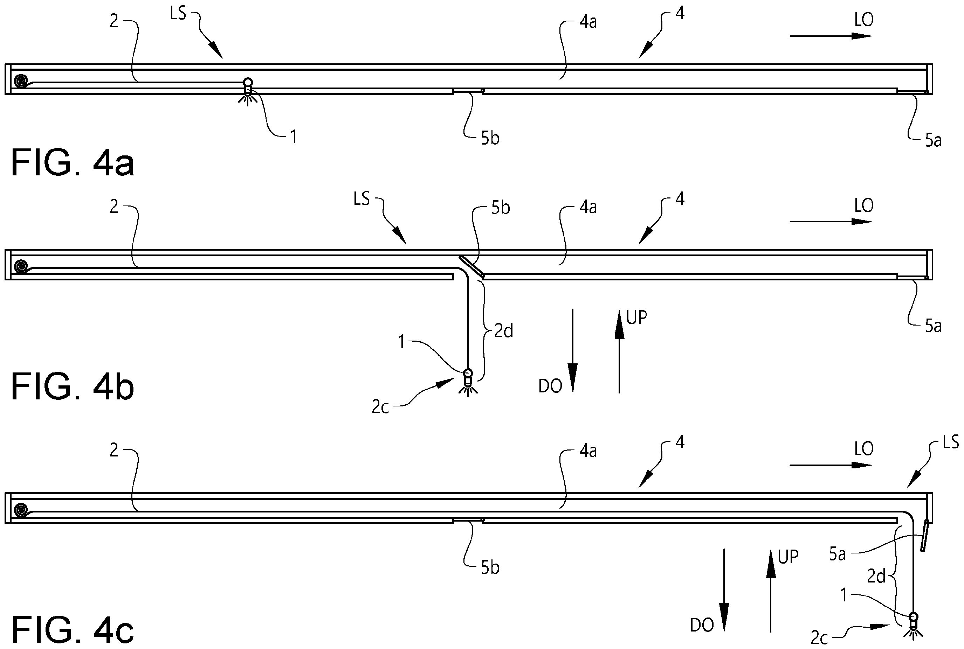

In an alternative embodiment of the lighting system LS illustrated in figures 3d-e and 4a-c, the track arrangement 4 adapted for guiding the interior sunscreen cover 2 upon movement has a different configuration. In this embodiment, the track arrangement 4 comprises guiding members 5 adapted for directing the interior sunscreen cover 2 in a downwards direction DO. By the guiding members 5, a leading edge section 2d of the interior sunscreen cover 2 including the leading edge 2c can be displaced into a downwards direction DO away from the track structure 4, as shown in figures 3d-e and 4b-c. In this way, the light source 1 is displaced in the downwards direction DO for an efficient disinfecting operation.

In the embodiment illustrated in figure 3d, each of the first track structure 4a and second track structure 4b is arranged with a guiding member 5. The guiding member 5 is positioned in a front section of each track structure, as understood from the figure. With this configuration, the lighting system LS may be operated as described above in connection to figures 3a-c, but the light source 1 can in addition also be displaced in the downwards direction DO, for example to emit light onto the interior surfaces S between front vehicle seats and the dashboard. To displace the light source 1 in the downwards direction DO, the interior sunscreen cover 2 is displaced beyond the closed position P_{CL} shown in figure 3c, and the leading edge section 2d of the interior sunscreen cover 2 with the light source 1 is allowed to fall down from the track structure 4 via the guiding member 5 into the position shown in figure 3d. The guiding members 5 may for example be arranged as openings in lower section of the first track structure 4a and the second track structure 4b respectively, allowing the displacement of the leading edge section 2d and the light source 1 in the downwards direction DO away from the track structure 4. When the light source 1 has been displaced a desired distance in the downwards direction DO, the movement of the interior sunscreen cover 2 may be stopped or alternatively the leading edge section 2d and the light source 1 of the interior sunscreen cover 2 may be displaced in an upwards direction UP. In this way, the leading edge section 2d and the light source 1 of the interior sunscreen cover 2 is displaced in the upwards direction UP after being displaced in the downwards direction DO for returning the leading edge 2c to the track structure 4. The light source 1 may if suitable be displaced in the downwards and upwards repeatedly in a reciprocating manner for an efficient disinfecting operation. The light source 1 may further be stopped in any suitable position.

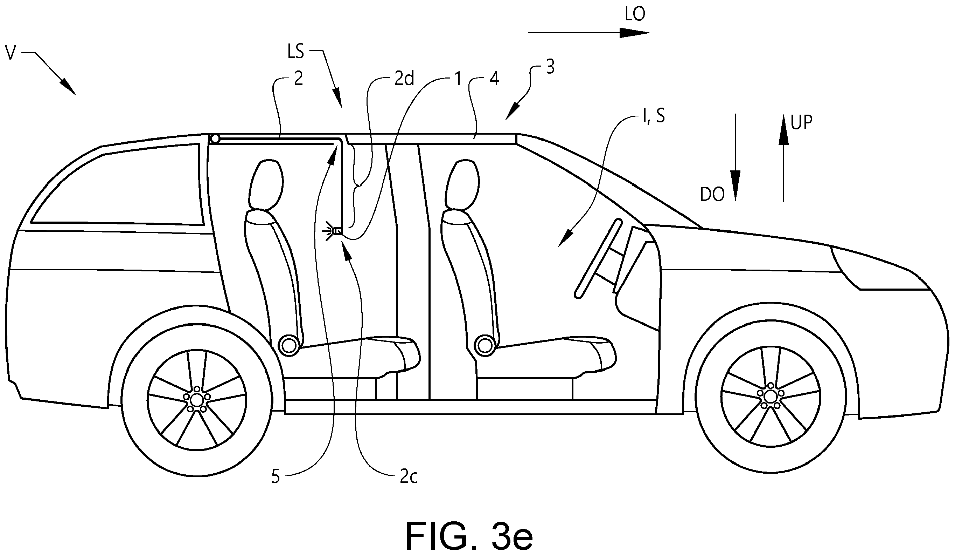

In the embodiment illustrated in figure 3e, each of the first track structure 4a and second track structure 4b is arranged with a guiding member 5. The guiding member 5 is positioned in a mid-section of each track structure, as understood from the figure. With this configuration, the lighting system LS may be operated as described above in connection to figures 3a-c, but the light source 1 can in addition also be displaced in the downwards direction DO, for example to emit light onto the interior surfaces S between front vehicle seats and rear vehicle seats. To displace the light source 1 in the downwards direction DO, the interior sunscreen cover 2 is displaced from a rear position forwards in the longitudinal vehicle direction LO, and the leading edge section 2d of the interior sunscreen cover 2 with the light source 1 is allowed to fall down from the track structure 4 via the guiding member 5 into the position shown in figure 3e. The guiding members 5 may for example be arranged as openings in the lower section of the first track structure 4a and the second track structure 4b respectively, allowing the displacement of the leading edge section 2d and the light source 1 in the downwards direction DO away from the track structure 4. When the light source 1 has been displaced a desired distance in the downwards direction DO, the movement of the interior sunscreen cover 2 may be stopped or alternatively the leading edge section 2d and the light source 1 of the interior sunscreen cover 2 may be displaced in an upwards direction UP. In this way, the leading edge section 2d and the light source 1 of the interior sunscreen cover 2 is displaced in the upwards direction UP after being displaced in the downwards direction DO for returning the leading edge 2c to the track structure 4. The light source 1 may if suitable be displaced in the downwards and upwards repeatedly in a reciprocating manner for an efficient disinfecting operation. The light source 1 may further be stopped in any suitable position.

In a further embodiment illustrated in figures 4a-c, the track arrangement 4 may be provided with first guiding members 5a positioned in the front section and second guiding members 5b positioned in the mid-section of each of the first track structure 4a and second track structure 4b. With this configuration, the first guiding members 5a and second guiding members 5b are for example arranged as pivoting shutter or door structures that can be opened or closed via a hinge or other suitable pivoting arrangement. In figure 4a, the first guiding members 5a and second guiding members 5b are arranged in a closed state preventing displacement of the light source in the downwards direction DO. With this positioning of the first guiding members 5a and second guiding members 5b the lighting system may be operated as described in connection to figures 3a-c above. In figure 4b, the first guiding members 5a are arranged in a closed state and the second guiding members 5b are arranged in an open state, and with this configuration, the light source 1 can be displaced at the mid-section of the track arrangement 4 in the downwards direction DO in a similar way as described in connection to figure 3e above. In figure 4c, the first guiding members 5a are arranged in an open state and the second guiding members 5b are arranged in a closed state, and with this configuration, the light source 1 can be displaced at the front section of the track arrangement 4 in the downwards direction DO in a similar way as described in connection to figure 3d above. The opening and closing of the respective guiding members may be controlled by the control unit 6 and the guiding members may be connected to suitable actuators, such as for example electric motors for the opening and closing operations.

It should be understood that the track arrangement could be provided with further guiding members or that the guiding members may be positioned differently. The guiding members 5 may have any suitable design and configuration for allowing the downwards and upwards movements of the interior sunscreen cover 2.

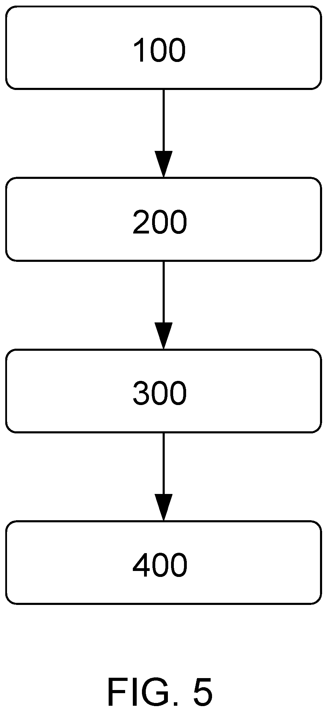

A flowchart of an exemplified method for operating an interior vehicle lighting system LS for providing a disinfecting effect to interior surfaces S of the vehicle V is shown in figure 5. The lighting system LS comprises at least one light source 1 attached to an interior sunscreen cover 2, where the interior sunscreen cover 2 is movably arranged relative to a sunroof 3 of the vehicle V, as described in the different embodiments above. The exemplified method comprises the steps described below with reference to figure 5.

In step 100, an activating command AC for moving the interior sunscreen cover 2 is initiated.

In step 200, the at least one light source 1 is activated upon initiation of the activation command AC. The at least one light source 1 may comprise a UV emitting lamp that is configured for emitting UV light to the interior surfaces S of the vehicle V.

In step 300, the at least one light source 1 is displaced relative to the interior surfaces S of the vehicle V upon movement of the interior sunscreen cover 2 upon initiation of the activating command AC, and light with a disinfecting effect is emitted to the interior surfaces S of the vehicle V with the at least one light source 1. Upon initiation of the activating command AC, the at least one light source 1 is displaced with the interior sunscreen cover 2 from an initial position P_{INI}. As described above, the vehicle lighting system may LS further comprise a track arrangement 4 adapted for guiding the interior sunscreen cover 2 upon movement, where the interior sunscreen cover 2 comprises a leading edge 2c and a leading edge section 2d including the leading edge 2c. The at least one light source 1 is attached to the interior sunscreen cover 2 in connection to the leading edge 2c. The track arrangement 4 may in an alternative embodiment further comprise guiding members 5, and with this configuration, step 300 may further include displacement of the leading edge section 2d and the at least one light source 1 in a downwards direction DO away from the track structure 4 by means of the guiding members 5, and displacement of the leading edge section 2d and the at least one light source 1 of the interior sunscreen cover 2 in an upwards direction UP after displacing the leading edge section 2d and the at least one light source 1 in the downwards direction DO for returning the leading edge 2c to the track structure 4.

In step 400, the displacement of the at least one light source 1 with the interior sunscreen cover 2 is stopped upon initiation of a stopping command SC. In this way, the at least one light source 1 is displaced with the interior sunscreen cover 2 from the initial position P_{INI} to at least one stopping position P_{STOP}. The stopping command SC may be initiated after a predetermined time duration.

It will be appreciated that the above description is merely exemplary in nature and is not intended to limit the present disclosure, its application or uses. While specific examples have been described in the specification and illustrated in the drawings, it will be understood by those of ordinary skill in the art that various changes may be made and equivalents may be substituted for elements thereof without departing from the scope of the present disclosure as defined in the claims. Furthermore, modifications may be made to adapt a particular situation or material to the teachings of the present disclosure without departing from the essential scope thereof. Therefore, it is intended that the present disclosure not be limited to the particular examples illustrated by the drawings and described in the specification as the best mode presently contemplated for carrying out the teachings of the present disclosure, but that the scope of the present disclosure will include any embodiments falling within the foregoing description and the appended claims. Reference signs mentioned in the claims should not be seen as limiting the extent of the matter protected by the claims, and their sole function is to make claims easier to understand.

### REFERENCE SIGNS

- 1:: Light source
- 2:: Interior sunscreen cover
- 2a:: First lateral side
- 2b:: Second lateral side
- 2c:: Leading edge
- 2d:: Leading edge section
- 3:: Sunroof
- 4:: Track arrangement
- 4a:: First track structure
- 4b:: Second track structure
- 5:: Guiding member
- 5a:: First guiding member
- 5b:: Second guiding member
- 6:: Control unit

- AC:: Activating command
- DO:: Downwards direction
- I:: Interior compartment
- LA:: Lateral vehicle direction
- LO:: Longitudinal vehicle direction
- LS:: Vehicle lighting system
- P_{INI}:: Initial position
- P_{STOP}:: Stopping position
- P_{CL}:: Closed position
- P_{FO}:: Fully open position
- P_{PO}:: Partly open position
- S:: Interior surface
- UP:: Upwards direction
- V:: Vehicle

## Claims

1. An interior vehicle lighting system (LS) providing a disinfecting effect to interior surfaces (S) of a vehicle (V), wherein the lighting system (LS) comprises at least one light source (1) attached to an interior sunscreen cover (2),
**characterized in that** the at least one light source (1) is adapted to emit light with a disinfecting effect to the interior surfaces (S) of the vehicle (V), wherein the interior sunscreen cover (2) is movably arranged relative to a sunroof (3) of the vehicle (V), wherein the at least one light source (1) is arranged for being displaced relative to the interior surfaces (S) of the vehicle (V) upon movement of the interior sunscreen cover (2).

2. The interior vehicle lighting system (LS) according to claim 1,
wherein the at least one light source (1) comprises a UV emitting lamp, wherein the at least one light source (1) is adapted to emit UV light to the interior surfaces (S) of the vehicle (V).

3. The interior vehicle lighting system (LS) according to claim 1 or 2,
wherein the at least one light source (1) is forming an integrated structural part of the interior sunscreen cover (2).

4. The interior vehicle lighting system (LS) according to any preceding claim,
wherein the vehicle lighting system (LS) further comprises a track arrangement (4), wherein the track arrangement (4) is adapted for guiding the interior sunscreen cover (2) upon movement.

5. The interior vehicle lighting system (LS) according to claim 4,
wherein the interior sunscreen cover (2) comprises a leading edge (2c), wherein the at least one light source (1) is attached to the interior sunscreen cover (2) in connection to the leading edge (2c), wherein the track arrangement (4) comprises guiding members (5) adapted for directing a leading edge section (2d) of the interior sunscreen cover (2) including the leading edge (2c) into a downwards direction (DO) away from the track structure (4), wherein the at least one light source (1) is displaced in the downwards direction (DO).

6. The interior vehicle lighting system (LS) according to claim 5,
wherein the leading edge (2c) is extending in a lateral vehicle direction (LA).

7. A method for operating an interior vehicle lighting system (LS) providing a disinfecting effect to interior surfaces (S) of a vehicle (V), wherein the lighting system (LS) comprises at least one light source (1) attached to an interior sunscreen cover (2), wherein the interior sunscreen cover (2) is movably arranged relative to a sunroof (3) of the vehicle (V), wherein the method comprises the steps:
activating the at least one light source (1); displacing the at least one light source (1) relative to the interior surfaces (S) of the vehicle (V) upon movement of the interior sunscreen cover (2); and emitting light with a disinfecting effect to the interior surfaces (S) of the vehicle (V) with the at least one light source (1).

8. The method according to claim 7,
wherein the at least one light source (1) comprises a UV emitting lamp, wherein the method further comprises the step: emitting UV light to the interior surfaces (S) of the vehicle (V) with the at least one light source (1).

9. The method according to claim 7 or 8,
wherein the method further comprises the steps: initiating an activating command (AC) for moving the interior sunscreen cover (2), wherein upon initiation of the activating command (AC), the at least one light source (1) is displaced with the interior sunscreen cover (2) from an initial position (P_{INI}).

10. The method according to claim 9,
wherein the method further comprises the step: displacing the at least one light source (1) with the interior sunscreen cover (2) from the initial position (P_{INI}) to at least one stopping position (P_{STOP}).

11. The method according to claim 9 or 10,
wherein the method further comprises the steps: displacing the at least one light source (1) with the interior sunscreen cover (2) from the initial position (P_{INI}) upon initiation of the activation command (AC), and stop the displacement of the at least one light source (1) with the interior sunscreen cover (2) upon initiation of a stopping command (SC).

12. The method according to claim 11,
wherein the stopping command (SC) is initiated after a predetermined time duration.

13. The method according to any of claims 9 to 12,
wherein the at least one light source (1) is moving from the initial position (P_{INI}) in a longitudinal vehicle direction (LO).

14. The method according to any of claims 7 to 13,
wherein the vehicle lighting system (LS) further comprises a track arrangement (4) adapted for guiding the interior sunscreen cover (2) upon movement, wherein the interior sunscreen cover (2) comprises a leading edge (2c) and a leading edge section (2d) including the leading edge (2c), wherein the at least one light source (1) is attached to the interior sunscreen cover (2) in connection to the leading edge (2c), wherein the track arrangement (4) comprises guiding members (5), wherein the method further comprises the step: displacing the leading edge section (2d) and the at least one light source (1) in a downwards direction (DO) away from the track structure (4) by means of the guiding members (5).

15. The method according to claim 14,
wherein the method further comprises the step: displacing the leading edge section (2d) and the at least one light source (1) of the interior sunscreen cover (2) in an upwards direction (UP) after displacing the leading edge section (2d) and the at least one light source (1) in the downwards direction (DO) for returning the leading edge (2c) to the track structure (4).
